# EUROPEAN PATENT APPLICATION

(11) **EP 1 790 294 A1**
(43) Date of publication of application: **30.05.2007**
(21) Application number: 05776997.8
(22) Date of filing: 05.09.2005
(51) Int. Cl.: A61B 17/10, B25C 5/11, B25C 5/16

(54) **STAPLER FOR MEDICAL APPLICATION**

(30) Priority: 15.09.2004 JP 2004268883
(71) Applicant: MAX CO., LTD., Chuo-ku, Tokyo 103-8502 (JP)
(72) Inventor: HIRANUMA, Toshio, ... (JP); HASHIMOTO, Masahiko, ... (JP); YAMAGUCHI, Shigenori, ... (JP)
(74) Representative: Samson & Partner
(86) International application number: PCT/JP2005/016257
(87) International publication number: WO 2006/030660

(57) **Abstract**

A medical stapler is provided with a sterilized cartridge removably attached to a stapler body 1 and accommodating a predetermined number of staples 9, a driving mechanism for driving a staple 9 supplied to a driving section at the tip of the cartridge, a manipulating lever 2 for actuating the driving mechanism and a counter device 20. The counter device 20 counts the number of staples 9 driven from the driving mechanism and indicates the counted result.

## Description

### Technical Field:

The present invention relates to a medical stapler employed to sew up a wound in a surgical operation.

### Background Art:

Generally, after a tissue of a living body has been incised in a surgical operation, a wound is sewed up using a sewing thread. For the reasons that an operation trace is not conspicuous and the healing is rapid, a system of sewing up using a staple is also adopted.

A medical stapler is provided with a stapler body including a driving mechanism and a manipulating lever for actuating the driving mechanism. By rotating the manipulating lever, staples are pushed out one by one and the staple is put into skin while it is being bent. Thus, after the skin has been sewed up, the staple is removed from the stapler body.

Meanwhile, in the medical stapler, the staple itself as well as the staplerbodymustbe sterilizedpreviously. Further, in a scene of medical treatment, in order to prevent such a medical accident that the staple is erroneously left in the living body during a sewing operation, the use of the staple is started in a state where a predetermined number of staples are loaded beforehand, and a number of the staples left in the medical stapler and a number of the staples actually employed in a sewing region are calculated. Whether or not a total of the numbers of both the above staples agrees with the number of the staples first loaded is always confirmed by visual calculation.

Incidentally, there are various kinds of staplers having the loaded number of staples up to 35, e.g. 10 staples or 20 staples according to the size of the wound.

In view of the above circumstance, the medical stapler is used with a predetermined number of staples being loaded. The medical stapler is roughly classified into an integral type incapable of separating components and a separating type capable of replacing a head having a predetermined number of staples. Either type of stapler is disposable as a rule for a sanitary reason. Namely, after it has been once employed for the operation, it is disposed (except the case where there are plural wounds).

Meanwhile, where the number of staples is counted, the number of spent staples can be easily counted. This is because the interval of sewing is about 1 cm and so the staples are sufficiently apart from one another. On the other hand, where the number of staples remaining within the stapler is counted, the width of the staple is very small and the staples reside close to one another in a magazine. So it is difficult to read the number of staples exactly. In order to obviate such inconvenience, there is also proposed a stapler in which with a staple feeding path within the stapler being transparent, numerals such as "5", "10" and "15" are indicated and the number of staples remaining in the staple feeding path can be known exactly to a degree.

Further, JP-U-05-093771 and JP-U-60-178585 disclose a document stapler equipped with a counter for the staples having been driven.

However, these staplers are directedto stapling a document. The document stapler disclosed in JP-U-05-093771 is not clear in the concrete structure. The document stapler disclosed in JP-U-60-178585 is complicate in structure and so not practical. Further, these document staplers use coupled staples and do not entirely take pollution due to a bacterium into consideration.

Where the medical stapler is used, it is necessary to exactly know not only the number of staples actually employed but also the remaining number of staples within the stapler. The reasons therefor are as follows. What number of staples is required is determined according to the length of the wound. For example, although ten staples are actually required, if the remaining number of staples is nine, the stapler now being used must be replaced by a new stapler because of shortage of one staple. After the one staple has been used, the new stapler will be disposed unless there is another wound requiring sewing. In this case, if it is known beforehand that the reaming number of staples is nine, the wound can be sewed up by nine staples by adjusting the interval of sewing. Further, where there are plural wounds, when another wound is sewed up using the same stapler immediately after one wound has been sewed up, if the remaining number of staples is unknown, it is not possible to determine whether or not sufficient sewing can be realized using the same stapler.

In the stapler equipped with a counter for adding up spent staples, what number of staples remains must be known by visual calculation.

### Disclosure of the Invention

One or more embodiments of the present invention provide a medical stapler capable of knowing the number of staples already used or the number of staples remaining within the stapler at a glance in a simple structure.

In accordance with one or more embodiments of the present invention, a medical stapler is provided with: a sterilized cartridge removably attached to a stapler body and accommodating a predetermined number of staples; a driving mechanism for driving a staple supplied to a driving section at the tip of the cartridge; a manipulating lever for actuating the driving mechanism; and a counter device for counting the number of staples driven from the driving mechanism and indicating the counted result.

For this reason, the number of staples already used can be known in a simple structure.

In accordance with one or more embodiments of the present invention, the counter device counts up the number of staples driven from the driving mechanism.

In accordance with one or more embodiments of the present invention, the counter subtracts the number of stapled driven from the driving mechanism thereby to indicate the remaining number of staples counted down in the cartridge.

By subtracting the number of stapled driven from the driving mechanism thereby to know the number of staples remaining in the cartridge, sewing-up of a wound can be effectively advanced.

In accordance with one or more embodiments of the present invention, the counter device is provided with an indictor disc which rotates in synchronism with the operation of the manipulating lever, and a numeral is successively indicated on the peripheral surface of the indicator disc so that it changes by one whenever the manipulating lever is manipulated.

For this reason, the number of staples already used or the remaining number of staples in the cartridge can be known in a simple structure.

In accordance with one or more embodiments of the present invention, the counter device digitally indicates the number of staples driven from the driving mechanism.

Since the number of staples is electronically counted, the number of components can be reduced.
Other aspects and advantages of the invention will be apparent from the following description and the appended claims.

### Brief description of the drawings:

[Fig. 1] Fig. 1 is a perspective view of a medical stapler according to the present invention.
[Fig. 2] Fig. 2 is an enlarged longitudinal sectional view of the above stapler.
[Fig. 3] Fig. 3 is a longitudinal sectional view of the main part showing the operating manner of the above stapler.
[Fig. 4] Fig. 4 is a longitudinal sectional view of the main part showing the operating manner of the above stapler.
[Fig. 5] Fig. 5 is a longitudinal sectional view of the main part showing the operating manner of the above stapler.
[Fig. 6(a)] Fig. 6(a) is a view for explaining the bending manner of a staple, which shows the state before driving.
[Fig. 6(b)] Fig. 6(b) is a view for explaining the bending manner of a staple, which shows the state during driving.
[Fig. 6(c)] Fig. 6(c) is a view for explaining the bending manner of a staple, which shows the state after driving.
[Fig. 7] Fig. 7 is a plan view of a see-through window in Fig. 2.
[Fig. 8] Fig. 8 is a longitudinal sectional view of the operating state of a counter device.
[Fig. 9] Fig. 9 is a sectional view of another example of the counter.
[Fig. 10] Fig. 10 is a plan view of the see-through window in Fig. 9.
[Fig. 11] Fig. 11 is a sectional view of the main part of still another example of the counter device.

### Description of Reference Numerals and Signs

- 1: stapler body
- 2: manipulating lever
- 9: staple
- 20: counter device

### Best Mode for Carrying Out the Invention:

Now referring to the drawings, an explanation will be given of various embodiments of the present invention.

### Embodiment 1

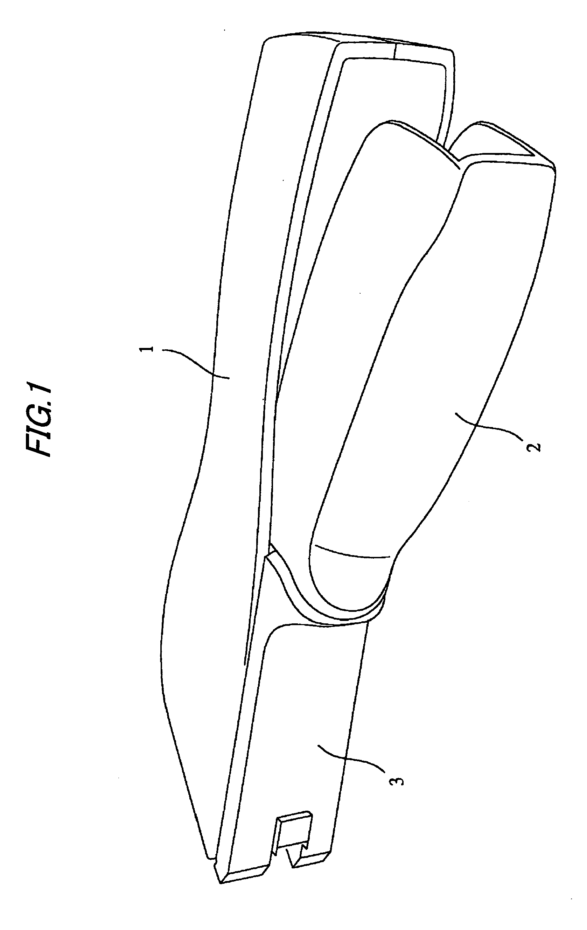

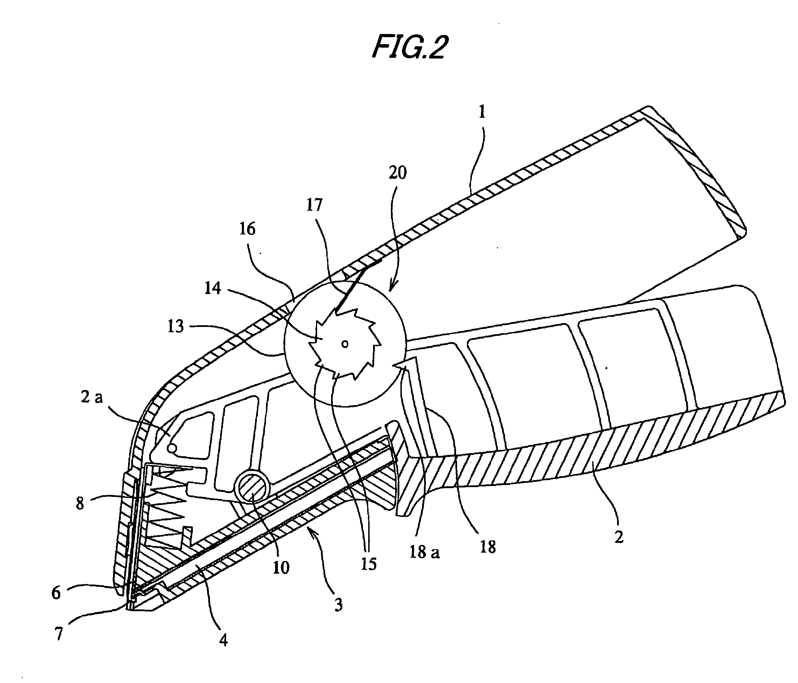

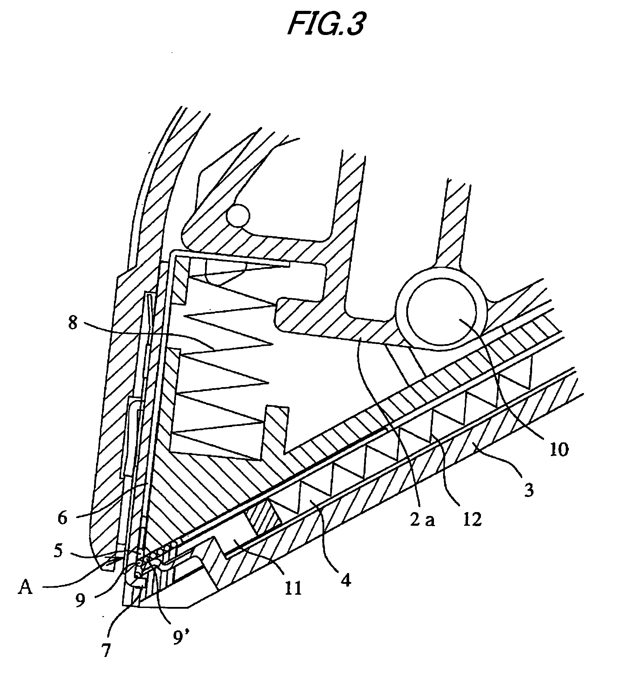

Fig. 1 is a perspective view of an embodiment of the present invention. Fig. 2 is an enlarged longitudinal sectional view of the above stapler. Fig. 3 is a longitudinal sectional view of the front part of the above stapler. In the drawings, reference numeral 1 denotes a stapler body, and 2 denotes a manipulating lever.

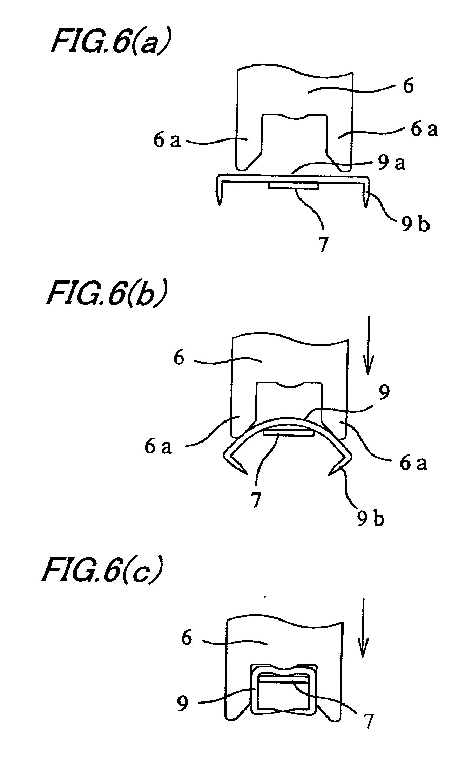

At the internal tip of the stapler body 1, a staple feeding path 4 and a driving mechanism A are provided. Above a driving section 5 formed at the front end of the staple feeding path 4, a driver plate 6 is arranged vertically slidably. Below the driving section 5, an anvil 7 is formed. The driver plate 6 is urged by a spring 8 so that it is always located at an initial position. Incidentally, as seen from Fig. 6(a), the lower end of the driver plate 6 is formed in an inverted concave shape having projecting pieces 6a formed on both sides. The staples 9 within the staple feeding path 4 are can be always supplied to the driving section 5 by a pusher 11 and a spring 12.

Next, the manipulating lever 2 is attached reciprocally pivotally on a supporting shaft 10 attached to the stapler body 1. Its front end is arranged to be contactable to the upper end of the driver plate 6 during forward movement.

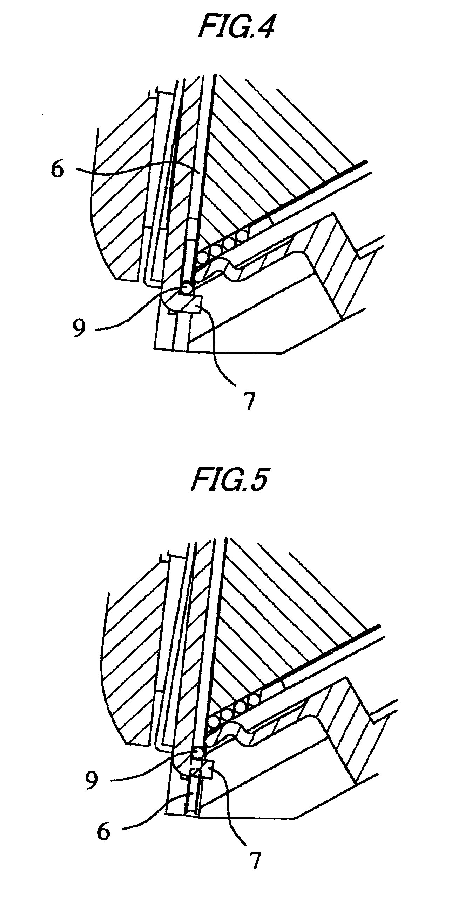

An explanation will be given of the operating manner of the driving mechanism A. If the manipulating lever is pulled to pivot in the one direction (forward movement), as seen from Figs.3 and 4, the driver plate 6 is pressed downward by the front end 2a of the manipulating lever 2. Thus, the driver plate 6 moves against spring force to push out the staple 9 supplied to the driving section 5. The center of a crown 9a of the staple 9 on the way of push-out is supported by the anvil 7 and cannot further move vertically. On the other hand, the projecting pieces 6a formed on both sides of the driver plate 6 press both sides of the crown 9a of the staple 9. Thus, as seen from Figs. 6(b) and 6(c), only both sides of the staple 9 are pressed down. As a result, both legs 9b of the staple 9 gradually bend around both ends of the anvil 7 so as to approach each other. The manipulating lever 2 is moved to a moving end, which results in the state as shown in Fig. 5. Thereafter, if the staple 9 is released from the driver plate 6 and anvil 7 by canceling force for the manipulating lever 2 to move backward, the sewing-up of a point of the wound is completed. By repeating the same manipulation, sewing-up of the entire wound is completed.

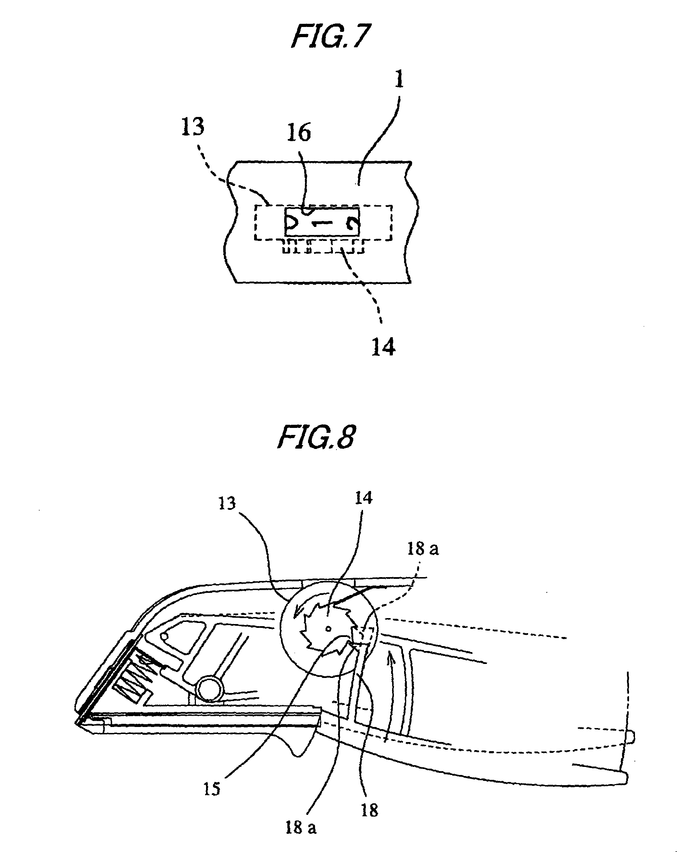

Next, as seen from Figs. 2 and 7, the above stapler body 1 includes a counter device 20 for counting the number of staples 9 driven from the driving mechanism. The counter device 20 uses the fact that a single staple 9 is driven whenever the manipulating lever 2 is pulled. The counter device 20 includes a latchet 14 and an indicator disc 13 attached to the stapler body 1 and the manipulating lever 2.

Specifically, within the stapler body 1, the latchet 14 is arranged rotatably. On the peripheral edge of the latchet 14, ten (may be fewer or more than ten) sawtooth teeth 15 are formed. The indicator disc 13 is secured to the side portion of the latchet 14, and on the outer peripheral face thereof, numerals are indicated. Further, on the upper face of the staple body 1, a transparent window 16 is formed in an area corresponding to the numerals. Incidentally, in order that the indicator disc 13 moves only unidirectionally, a detent 17 formed of a metallic spring is attached to the stapler body 1.

On the other hand, an actuating piece 18 is protruded from the manipulating lever 2. When the manipulating lever 2 is pulled, on the way thereof, a tip securing segment 18a of the actuating piece 18 is engaged with the teeth 15 of the latchet 14. Thus, as indicated in dotted line in the drawing, when the manipulating lever 2 is moved to the moving end, the teeth 15 are also pushed up. In this case, the degree of movement is set so as to correspond to one of the teeth.

In accordance with the above configuration, whenever the manipulating lever 2 is pulled up, one of the teeth 15 of the latchet 14 is rotated. Thus, the numeral counted up or counted down by one is indicated on the indicator disc 13. By seeing this numeral through the transparent window 16, the number of staples 9 can be confirmed.

Incidentally, if the numerals on the indicator disc 13 are successively indicated like '1', '2', '3', '4' ... from '0' along the rotating direction of the latchet 14, whenever the manipulating lever 2 is pulled up, the numeral indicated on the indicator disc 13 is also counted up by one. On the other hand, if the numerals on the indicator disc 13 are successively indicated like, for example, '9', '8', '7', '6' ... from '0', whenever the manipulating lever 2 is pulled up, the numeral indicated on the indicator disc 13 is also counted down by one. The numeral counted down on the indicated disc 13 indicates the remaining number of staples 9 in the cartridge.

In this way, by subtracting the number of staples 9 driven in order to know the number of staples 9 remaining in the cartridge, sewing-up of the wound can be effectively advanced.

### Embodiment 2

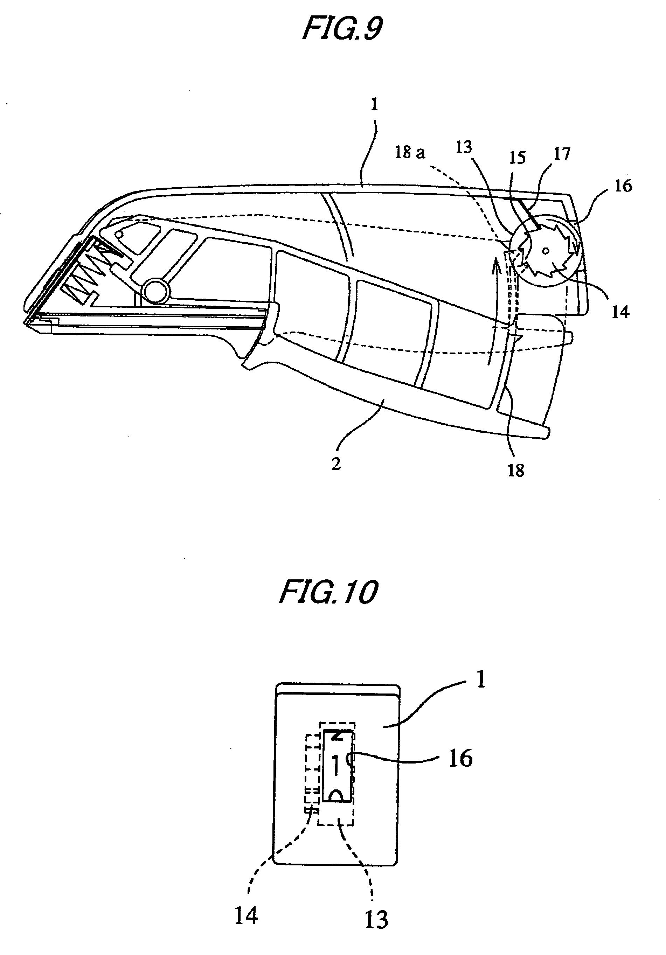

Figs. 9 and 10 show another embodiment. In this embodiment, the latchet 14 is arranged on the rear side of the stapler body 1 and the actuating piece 18 is also arranged on the rear side of the manipulating lever 2. Further, the detent 17 is provided on the back side of the upper face of the stapler body 1. The transparent window 16 is formed on the rear side of the stapler body 1.

In accordance with the above configuration also, whenever the manipulating lever 2 is pulled up, one of the teeth 15 of the latchet 14 is rotated. Correspondingly, the numeral counted up by one is indicated on the indicator disc 13. By seeing this numeral through the transparent window 16, the number of staples 9 already used for sewing-up can be confirmed.

### Embodiment 3

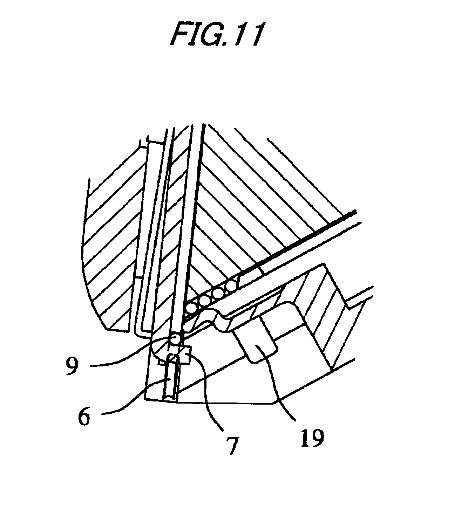

Fig. 11 shows a mechanism for electronically counting the number of staples 9 in which a sensor 19 for detecting the staple 9 is arranged behind the anvil 7 in the driving section of the stapler body 1 and the staple 9 exhausted after having been driven and bent is detected for counting. The counted result may be indicated on the indicator disc 13.
The present invention has been explained in detail and referring to the specific embodiment. However, it is apparent to those skilled in the art that the present invention can be changed or modified in various manners without departing from the spirit and scope of the invention.

This application is based on Japanese Patent Application (Patent Application No. 2004-268883) filed on September 15, 2004, and the contents of which are incorporated herein by reference.

### Industrial Applicability:

In accordance with one or mode embodiments of the present invention, it is possible to know the number of staples already used or the number of staples remaining within the stapler at a glance in a simple structure.

## Claims

1. A medical stapler comprising:
a cartridge removably attached to a stapler body and accommodating a predetermined number of staples;
a driving mechanism for driving a staple supplied to a driving section at the tip of the cartridge;
a manipulating lever for actuating the driving mechanism; and
a counter device for counting and indicating a number of staples driven from the driving mechanism.

2. The medical stapler according to claim 1, wherein the cartridge is previously sterilized.

3. The medical stapler according to claim 1, wherein the counter device counts up the number of staples driven from the driving mechanism.

4. The medical stapler according to claim 1, wherein the counter subtracts the number of stapled driven from the driving mechanism thereby to indicate the remaining number of staples counted down in the cartridge.

5. The medical stapler according to claim 1, wherein the counter device comprises an indictor disc that rotates in synchronism with an operation of the manipulating lever, and
a numeral is successively indicated on a peripheral surface of the indicator disc so that the numeral changes by one whenever the manipulating lever is operated.

6. The medical stapler according to claim 1, wherein the counter device digitally indicates the number of staples driven from the driving mechanism.
